**Europäisches Patentamt**

**European Patent Office**

**Office européen des brevets**

(11) Numéro de publication : **0 406 041 B1**

(12)

# FASCICULE DE BREVET EUROPEEN

(45) Date de publication du fascicule du brevet :
**08.09.93 Bulletin 93/36**

(51) Int. Cl.⁵ : **A23K 1/16,** A23K 1/18,
A61K 9/52

(21) Numéro de dépôt : **90401566.6**

(22) Date de dépôt : **08.06.90**

(54) **Utilisation de compositions dégradables par voie enzymatique pour l'enrobage d'additifs alimentaires destinés aux ruminants.**

Le dossier contient des informations techniques présentées postérieurement au dépôt de la demande et ne figurant pas dans le présent fascicule.

(30) Priorité : **12.06.89 FR 8907717**

(43) Date de publication de la demande :
**02.01.91 Bulletin 91/01**

(45) Mention de la délivrance du brevet :
**08.09.93 Bulletin 93/36**

(84) Etats contractants désignés :
**AT BE CH DE DK ES FR GB GR IT LI LU NL SE**

(56) Documents cités :
**EP-A- 0 188 953**
**EP-A- 0 268 533**
**EP-A- 0 321 337**

(56) Documents cités :
**US-A- 4 066 754**
**DERWENT FILE SUPPLIER WPI/L, 1966, AN=66-19131F (00), Derwent Publications Ltd, Londres, GB; & JP-B-65 025 516 (TAISHO PHARM. CO., LTD)**

(73) Titulaire : **RHONE-POULENC SANTE**
**20, avenue Raymond Aron**
**F-92160 Antony (FR)**

(72) Inventeur : **Ardaillon, Pierre**
**10 Impasse Beau-Vallon**
**F-69800 Saint-Priest (FR)**
Inventeur : **Franzoni, Christine**
**14 Quai de la Pêcherie**
**F-69001 Lyon (FR)**

(74) Mandataire : **Le Pennec, Magali et al**
**RHONE-POULENC SANTE Service Brevets Santé, 20 Avenue Raymond Aron**
**F-92160 Antony (FR)**

EP 0 406 041 B1

## Description

La présente invention concerne l'utilisation de compositions degradables par voie enzymatique pour l'enrobage d'additifs alimentaires destinés aux ruminants.

Les substances biologiquement actives administrées aux ruminants subissent généralement une dégradation au niveau du rumen et ne peuvent ainsi exercer pleinement leur activité lorsqu'elles progressent dans le tractus digestif. Il est donc nécessaire de protéger les substances biologiquement actives de telle manière qu'elles résistent à la dégradation dans le rumen.

Jusqu'à présent, il a été proposé d'enrober les substances biologiquement actives par des compositions qui présentent la propriété d'être stables dans le rumen dont le pH est voisin de 6 et de permettre la libération de la substance active dans la caillette dont le pH est voisin de 2.

Généralement, les compositions d'enrobage connues sont constituées de l'association d'une substance sensible aux variations du pH, choisie en particulier parmi des copolymères basiques synthétiques, avec des substances hydrophobes qui peuvent être choisies, par exemple, parmi les acides gras ou leurs dérivés et les polymères hydrophobes et elles forment une pellicule continue autour de la substance biologiquement active. De telles compositions sont décrites, par exemple, dans les brevets français FR 78 23966 (2 401 620), FR 78 23968 (2 401 621) ou FR 81 18954 (2 514 261).

La sélection des compositions permettant la réalisation du but recherché est effectuée sur la base de leur différence de comportement en fonction du pH. Le but recherché est atteint lorsque la composition d'enrobage est stable à un pH supérieur ou égal à 5,5 pendant une longue période (24 à 48 heures) et lorsqu'elle est dégradée rapidement (quelques minutes à quelques heures) à un pH inférieur ou égal à 3,5. Il en résulte que des compositions d'enrobage dont la différence de comportement à pH=6 ou à pH=2 est faible ne peuvent pas, a priori, être utilisables dans le but recherché. Ainsi, dans la demande de brevet européen EP 0 188 953 sont revendiquées des compositions d'enrobage qui sont constituées d'un liant filmogène non hydrosoluble dont l'hydrophilie est contrôlée et d'une substance sensible aux variations du pH. Les exemples comparatifs qui sont donnés montrent que, en l'absence du liant filmogène non hydrosoluble ou en l'absence de la substance sensible aux variations pH, on obtient des compositions qui sont a priori inutilisables dans le but recherché puisqu'elles ont des stabilités comparables à pH=6 et à pH=2.

Il a maintenant été trouvé, et c'est ce qui fait l'objet de la présente invention, qu'il est possible d'utiliser des compositions d'enrobage stables dans les conditions de pH du rumen et éventuellement en milieu plus acide pour enrober des substances actives destinées aux ruminants qui sont libérées non pas uniquement au niveau de la caillette mais aussi au niveau de l'intestin grêle tout en conservant leur activité bénéfique, la dégradation de l'enrobage étant effectuée par les enzymes présentes dans l'intestin et dans la caillette.

Selon la présente invention, une substance biologiquement active peut être enrobée par une composition :
- qui est stable dans le rumen, ce qui implique une bonne résistance à la flore microbienne et aux enzymes de ce milieu,
- qui est relativement stable dans la caillette dont le pH est acide, et
- qui est fortement dégradée au niveau de l'intestin grêle grâce aux enzymes rencontrées entre la caillette et l'iléon.

Les compositions d'enrobage utilisables selon l'invention sont constituées de zéine associée à une substance hydrophobe et éventuellement à un polymère non hydrosoluble et contenant au moins une charge minérale. Le polymère non hydrosoluble est choisi de préférence parmi les éthers ou les esters de cellulose non hydrosolubles tels que l'éthylcellulose, l'acétate de cellulose, le propionate de cellulose ou l'acétobutyrate de cellulose, et les esters polyvinyliques tels que l'acétate de polyvinyle. On préfère utiliser l'éthylcellulose. La substance hydrophobe a de préférence un point de fusion supérieur à 60°C, elle est choisie parmi les acides ras (acide stéarique, acide béhénique), les esters gras, les alcools gras et leurs mélanges. On préfère utiliser l'acide stéarique. La charge minérale est choisie notamment parmi les charges pH sensible ou non et notamment parmi le talc et/ou la silice et/ou les carbonates et/ou les polyphosphates complexes à base de $Na_2O$, $CaO$, $P_2O_5$ et $Al_2O_3$.

Selon une première préférence, la composition d'enrobage est constituée en poids de :
- 10 à 90 % de zéine,
- 0 à 80 % d'un polymère non hydrosoluble,
- 10 à 90 % d'une substance hydrophobe,
et contient 5 à 300 % de charge minérale calculée par rapport à la zéine plus la substance hydrophobe et éventuellement le polymère non hydrosoluble.

Selon une deuxième préférence, elle est constituée de :
- 30 à 80 % de zéine,
- 0 à 70 % d'un polymère non hydrosoluble,

- 10 à 70 % d'une substance hydrophobe,

et contient 5 à 150 % de charge minérale calculée par rapport à la zéine plus la substance hydrophobe et éventuellement le polymère non hydrosoluble.

Les compositions d'enrobage d'additifs alimentaires destinés à l'alimentation des ruminants peuvent contenir éventuellement en plus un ou plusieurs agents plastifiant. Ces agents plastifiants sont choisis parmi la triacétine, le phtalate de butyle, l'oéate de sodium, le propylène glycol.

Il est particulièrement important de remarquer que les compositions utilisables selon l'invention sont constituées de substances qui sont connues pour pouvoir être utilisées dans l'industrie alimentaire, ce qui apporte un avantage considérable sur les compositions antérieurement connues.

Les compositions selon l'invention sont particulièrement utiles pour enrober des substances actives qui sont sensibles à la dégradation dans le rumen.

Les compositions d'enrobage utilisables selon l'invention peuvent être obtenues en dispersant ou en dissolvant la zéine, la charge la substance hydrophobe et éventuellement le polymère non hydrosoluble, dans un solvant organique ou dans un mélange de solvants organiques convenables choisis en fonction de la nature spécifique des constituants. Généralement, la composition d'enrobage est obtenue après évaporation du ou des solvants.

Selon la présente invention, les compositions d'enrobage sont particulièrement utiles pour protéger des substances thérapeutiques ou nutritives diverses telles que des médicaments, des vitamines et/ou des acides aminés tels que la méthionine et/ou la lysine destinés à être administrés par voie orale à des ruminants. Les substances enrobées sont généralement mélangées à la nourriture des animaux.

Les substances enrobées sont généralement des granulés sous forme de microcapsules constituées d'un ou plusieurs noyaux entouré(s) d'une pellicule continue de la composition d'enrobage. En général, la composition d'enrobage représente 5 à 60 % en poids du granulé total.

Les granulés peuvent être obtenus par application des techniques connues. Selon la nature de la composition d'enrobage, on utilise soit des techniques d'extrusion ou de pulvérisation de solutions en lit fluidisé ou agité, soit des techniques d'encapsulation en milieu fondu ou semi-fondu, soit des techniques d'enrobage en milieu liquide telles que la coacervation.

La taille des granulés sera fonction de l'utilisation qui en est faite et plus particulièrement elle sera déterminée en fonction de l'animal auquel ils sont destinés.

Il est possible d'enrober des substances actives se présentant sous forme de granulés dont la taille est comprise entre 0,1 et 5 mm et de préférence entre 0,6 et 2 mm.

D'un intérêt tout particulier sont les granulés qui contiennent comme substances actives la méthionine et/ou la lysine et/ou des vitamines (vitamine A) dont le rôle est très important dans l'alimentation des animaux et plus spécialement des ruminants.

Les exemples suivants illustrent la présente invention.

EXEMPLES 1 A 4

Dans un mélange de 500 cm3 de dichlorométhane et de 500 cm3 d'éthanol absolu, on dissout 70 g de zéine, 45 g d'acide stéarique pur et 15 g d'éthylcellulose et la quantité de talc (stéanic 005) indiquée dans le tableau.

La suspension obtenue est pulvérisée, par la technique du "spray-coating" sur 350 g de méthionine sous forme de granulés dont la diamètre moyen est compris entre 0,63 et 0,8 mm.

Pour chaque composition utilisée, on détermine le titre en méthionine des granulés enrobés ainsi que le pourcentage de libération de la méthionine après 6 et 24 heures en milieu aqueux tamponné à pH=6 à 40°C.

Le relargage de la méthionine contenue dans les granulés obtenus est examiné, dans des conditions déterminées, en agitant une quantité connue de granulés dans un milieu tamponné maintenu à pH constant à une température de 40°C.

Les résultats sont reportés dans le tableau 1.

EXEMPLE 5

On reproduit l'exemple 1 en utilisant :
- 40 g de zéine F 4000
- 25 g d'acide stéarique
- 25 g d'éthylcellulose N22
- 10 g de carbonate de calcium
Le relargage de la méthionine contenue dans les granulés obtenus est examiné, dans les conditions déterminées, dans un milieu tamponné maintenu à pH constant (6 et 2) à une température de 40°C (voir tableau 2).

TABLEAU 1

| Exemples | Composition d'enrobage (parties en poids) | | | | quantité en poids de composition d'enrobage / granulé enrobé | Titre en Méthionine % | Pourcentage de méthionine libérée à pH6 et à 40°C | |
|---|---|---|---|---|---|---|---|---|
| | Zéine | Ethyl-cellulose | Acide stéarique | Talc | | | 6 H | 24 H |
| 1 | 54 | 35 | 11 | 0 | 20 | 78,7 | 3,2 | 7,3 |
| 2 | 50 | 32 | 10 | 8 | 19 | 79,0 | 2,7 | 6,0 |
| 3 | 47 | 30 | 10 | 13 | 20 | 78,6 | 3,8 | 8,3 |
| 4 | 44 | 28 | 9 | 19 | 19 | 79,0 | 3,0 | 7,0 |

4

EP 0 406 041 B1

**TABLEAU   2**

| Exemple | Composition d'enrobage (parties en poids) | | | | quantité de composition d'enrobage / granulé enrobé | Titre en Méthionine % | Pourcentage de méthionine libérée à 40°C | | | |
|---|---|---|---|---|---|---|---|---|---|---|
| | Zéine | Ethyl-cellulose | Acide stéarique | Talc | | | PH6 | | PH2 | |
| | | | | | | | 6H | 24H | 5H | 24H |
| 5 | 40 | 25 | 25 | 10 | 20 % | 78,5 | 2,0 | 10,0 | 2,0 | 16,0 |

**Revendications**

1. Utilisation d'une composition pour l'enrobage d'additifs alimentaires afin d'obtenir des granulés, destinés à l'alimentation des ruminants, qui sont stables dans la panse et dans la caillette et qui sont dégradés par les enzymes de l'intestin caractérisée en ce qu'elle est constituée de zéine associée à une substance hydrophobe choisie parmi les acides gras, les esters gras, les alcools gras et leurs mélanges et éventuellement un polymère non hydrosoluble et qu'elle contient en outre au moins une charge minérale.

2. Utilisation d'une composition selon la revendication 1 caractérisée en ce que la charge minérale est choisie parmi le talc la silice, le carbonate de sodium.

3. Utilisation d'une composition selon la revendication 1 caractérisé en ce qu'elle contient en poids :
   - 10 à 90 % de zéine,
   - 0 à 80 % d'un polymère non hydrosoluble,
   - 10 à 90 % d'une substance hydrophobe,
   et 5 à 300 % de charge minérale calculée par rapport à la zéine plus la substance hydrophobe et éventuellement le polymère hydrosoluble.

4. Utilisation d'une composition selon la revendication 1 caractérisé en ce qu'elle contient en poids :
   - 30 à 80 % de zéine,
   - 0 à 70 % d'un polymère non hydrosoluble,
   - 10 à 70 % d'une substance hydrophobe,
   et de 5 à 150 % en poids de charge minérale calculée par rapport au à la zéïne plus la substance hydrophobe et éventuellement le polymère hydrosoluble.

5. Utilisation selon l'une des revendications 1 à 4 caractérisé en ce que la substance hydrophobe a un point de fusion supérieur à 60°C.

6. Utilisation selon l'une des revendications 1 à 4 d'une composition dans laquelle le polymère non hydrosoluble est choisi parmi l'éthylcellulose, l'acétate de cellulose, le propionate de cellulose, l'acétobutyrate de cellulose et l'acétate de polyvinyle.

7. Utilisation selon l'une des revendications 1 à 4 pour l'enrobage d'additifs alimentaires choisis parmi les médicaments, les vitamines et les acides aminés.

8. Utilisation selon l'une des revendications 1 à 4 pour l'enrobage d'additifs alimentaires choisis parmi la méthionine et/ou la lysine.

9. Additifs alimentaires enrobés en utilisant une composition selon l'une des revendications 1 à 6.

10. Additifs selon la revendication 9 caractérisé en ce qu'ils sont constitués de 40 à 95 % d'additifs et de 5 à 60 % de composition selon l'une quelconque des revendications 1 à 6.

**Patentansprüche**

1. Verwendung einer Zusammensetzung für die Umhüllung von Futtermittelzusätzen zur Erzielung von Granulaten für das Futter von Wiederkäuern, die im Pansen und im Labmagen stabil sind und die durch die Enzyme des Darms abgebaut werden, dadurch gekennzeichnet, daß sie aus Zein in Assoziation mit einer hydrophoben Substanz, ausgewählt unter den Fettsäuren, den Fettestern, den Fettalkoholen und deren Gemischen, und gegebenenfalls einem nicht wasserlöslichen Polymeren besteht und daß sie außerdem zumindest einen mineralischen Füllstoff enthält.

2. Verwendung einer Zusammensetzung gemäß Anspruch 1, dadurch gekennzeichnet, daß der mineralische Füllstoff unter Talk, Siliciumdioxyd und Natriumcarbonat ausgewählt wird.

3. Verwendung einer Zusammensetzung gemäß Anspruch 1, dadurch gekennzeichnet, daß sie auf das Gewicht bezogen enthält:
   - 10 bis 90% Zein,

6

- 0 bis 80% eines nicht wasserlöslichen Polymeren,
- 10 bis 90% einer hydrophoben Substanz,

und 5 bis 300% mineralischen Füllstoff, berechnet in Bezug auf das Zein plus die hydrophobe Substanz und gegebenenfalls das wasserlösliche Polymere.

4. Verwendung einer Zusammensetzung gemäß Anspruch 1, dadurch gekennzeichnet, daß sie auf das Gewicht bezogen enthält:
- 30 bis 80% Zein,
- 0 bis 70% eines nicht wasserlöslichen Polymeren,
- 10 bis 70% einer hydrophoben Substanz,

und 5 bis 150 Gew.-% mineralischen Füllstoff, berechnet in Bezug auf das Zein plus die hydrophobe Substanz und gegebenenfalls das wasserlösliche Polymere.

5. Verwendung gemäß einem der Ansprüche 1 bis 4, dadurch gekennzeichnet, daß die hydrophobe Substanz einen Schmelzpunkt von höher als 60°C besitzt.

6. Verwendung gemäß einem der Ansprüche 1 bis 4 einer Zusammensetzung, worin das nicht wasserlösliche Polymere unter Ethylcellulose, Celluloseacetat, Cellulosepropionat, Celluloseacetobutyrat und Polyvinylacetat ausgewählt wird.

7. Verwendung gemäß einem der Ansprüche 1 bis 4 für die Umhüllung von Futtermittelzusätzen, ausgewählt unter den Arzneimitteln, den Vitaminen und den Aminosäuren.

8. Verwendung gemäß einem der Ansprüche 1 bis 4 für die Umhüllung von Futtermittelzusätzen, ausgewählt unter dem Methionin und/oder dem Lysin.

9. Unter Verwendung einer Zusammensetzung gemäß einem der Ansprüche 1 bis 6 umhüllte Futtermittelzusätze.

10. Zusätze gemäß Anspruch 9, dadurch gekennzeichnet, daß sie aus 40 bis 95% Zusätzen und 5 bis 60% der Zusammensetzung gemäß einem der Ansprüche 1 bis 6 bestehen.


**Claims**

1. Use of a composition for coating feed additives in order to obtain granules, intended for feeding ruminants, which are stable in the rumen and in the abomasum and which are degraded by intestinal enzymes, characterised in that it consists of zein combined with a hydrophobic substance chosen from fatty acids, fatty esters, fatty alcohols and mixtures thereof and, optionally, a water-insoluble polymer, and in that it additionally contains at least one inorganic filler.

2. Use of a composition according to Claim 1, characterised in that the inorganic filler is chosen from talc, silica and sodium carbonate.

3. Use of a composition according to Claim 1, characterised in that it contains, by weight:
- 10 to 90% of zein,
- 0 to 80% of a water-insoluble polymer,
- 10 to 90% of a hydrophobic substance,

and 5 to 300% of an inorganic filler calculated relative to zein plus the hydrophobic substance and, optionally, the water-insoluble polymer.

4. Use of a composition according to Claim 1, characterised in that it contains, by weight:
- 30 to 80% of zein,
- 0 to 70% of a water-insoluble polymer,
- 10 to 70% of a hydrophobic substance,

and 5 to 150% by weight of inorganic filter calculated relative to zein plus the hydrophobic substance and, optionally, the water-insoluble polymer.

5. Use according to one of Claims 1 to 4, characterised in that the hydrophobic substance has a melting point greater than 60°C.

6. Use according to one of Claims 1 to 4 of a composition in which the water-insoluble polymer. is chosen from ethyl cellulose, cellulose acetate, cellulose propionate, cellulose acetobutyrate and polyvinyl acetate.

7. Use according to one of Claims 1 to 4 for coating feed additives chosen from medicinal products, vitamins and amino acids.

8. Use according to one of Claims 1 to 4 for coating feed additives chosen from methionine and/or lysine.

9. Feed additives coated using a composition according to one of Claims 1 to 6.

10. Additives according to Claim 9, characterised in that they consist of 40 to 95% of additives and 5 to 60% of the composition according to any one of Claims 1 to 6.